# EUROPEAN PATENT SPECIFICATION

(11) **EP 2 599 515 B1**
(45) Date of publication and mention of the grant of the patent: **16.03.2016**
(21) Application number: 11191649.0
(22) Date of filing: 02.12.2011
(51) Int. Cl.: A61M 16/00, A61M 16/12, A61M 16/18, A61M 16/08

(54) **Gas delivery unit for delivering breathing gas for subject breathing and arrangement for maintaining vital functions of a subject**
Gasversorgungseinheit und Verfahren zum Liefern von Atemgas zur Beatmung eines Patienten und Anordnung zur Aufrechterhaltung der Vitalfunktionen eines Patienten
Unité de fourniture de gaz et procédé pour fournir un gaz respiratoire pour la respiration d'un sujet et agencement pour le maintien des fonctions vitales d'un sujet

(43) Date of publication of application: 05.06.2013
(73) Proprietor: General Electric Company, Schenectady, NY 12345 (US)
(72) Inventor: Häggblom, Tom, 01520 Vantaa (FI)
(74) Representative: Illingworth-Law, William Illingworth

(56) References cited:
- EP-A2- 0 916 357
- US-A- 5 049 317
- US-A1- 2006 196 505
- US-A1- 2011 197 889

## Description

### BACKGROUND OF THE INVENTION

This disclosure relates generally to a gas delivery unit and method for delivering breathing gas for subject breathing. The disclosure also relates to an arrangement for maintaining vital functions of a subject.

In order to perform surgery the patient must be anesthetized. The anesthesia is provided either as a local/regional anesthesia or general anesthesia. During local/regional anesthesia the patient is awake or sedated and needs often supplemental oxygen. Regional anesthesia is anesthesia affecting only a large part of the body, such as a limb or the lower half of the body. Regional anesthetic techniques can be divided into central and peripheral techniques. The central techniques include so called neuraxial blocks (epidural anesthesia, spinal anesthesia). The peripheral techniques can be further divided into plexus blocks such as brachial plexus blocks, and single nerve blocks.

Regional anesthesia may be performed as a single bolus or with a continuous catheter through which medication is given over a prolonged period of time, e.g. continuous peripheral nerve block. Regional anesthesia can be provided by injecting local anesthetics directly into the veins of an arm (provided the venous flow is impeded by a tourniquet). This is called intravenous regional technique. Regional anesthesia may provide anesthesia (absence of feeling, including pain) to allow a surgical operation, or provide post-operative pain relief. Various brachial plexus blocks exist for shoulder and arm procedures.

Local anesthesia is any technique to induce the absence of sensation in part of the body. Local anesthesia is provided to a small part of the body such as a tooth or an area of skin. Unlike general anaesthesia, patients may remain awake during the procedure when using local/regional anesthesia, resulting in reduced side-effects and enabling the surgeon to converse with the patient during the procedure if required. However, many patients prefer to receive sedation either during the local/regional anesthesia, the procedure, or both.

The general anesthesia incorporates hypnosis and pain prevention and is achieved with anesthetizing drugs. The most common drugs used for the purpose are inhalation anesthetics. These are either gases (N₂O) or volatile liquids (inhalation agents). Before delivery to patient breathing the inhalation agents are converted to gaseous form in anesthesia vaporizers. The breathing gas delivered to the patient is a mixture of oxygen (O₂), balance gas N₂O or N₂, and the volatile agent. Patient concentration for O₂ varies normally between 25-35 volume %, but may sometimes increase over 80%. The gas mixture including oxygen and a balance gas is mixed gas. The gas mixture including oxygen, balance gas and vaporized anesthetic agent is fresh gas. The anesthesia delivery systems may comprise multitude of vaporizers for different agents, or the vaporizer is easily exchangeable to another. The delivery systems are designed to allow delivery of one agent at a time, but for various reasons a need to change the agent from one to another may emerge. Such reasons are e.g. better acceptance of one agent when patient is still awake during anesthesia induction and faster recovery from anesthesia or less post-anesthesia nausea of another.

Once vaporized, the anesthetic agent is delivered in the fresh gas to anesthesia breathing circuit for delivery to patient. The anesthesia breathing system allows re-breathing of the patient exhaled breathing gas. This re-breathing is used to preserve patient expired expensive and environmental-hostile anesthesia agent vapors to reduce the agent consumption.

The re-breathing circuit comprises of inspiration- and expiration limbs, Y-piece, CO2 absorber to refresh the exhalation gas for re-breathing, and ventilator tubing. Other breathing gas volumes are the ventilator gas isolation system and patient lungs. The total gas volume of the system may be up to 7 liters.

The breathing gas meets the blood circulation in the lungs. There the agent dissolves into the blood that transports the agent to further body. From the blood the agent dissolves further to various tissues including the site of anesthetic effect in brains. Each of the agents has their own threshold brain concentration to be achieved in order to make the patient anesthetized. The required concentration is also patient specific, but for each agent a mean alveolar concentration (MAC) has been determined.

During local and regional anesthesia supplemental oxygen may be required for the patient, which is a reason to interrupt the anesthetic agent delivery introduced hereinbefore and supply only oxygen or oxygen-air mixture.

It is known to deliver through an integrated or independent device, comprising at least an auxiliary flow meter, 100% oxygen to a patient through a nasal cannula or face mask. The device is separate from typical gas delivery devices of the anesthesia machines used when delivering both the fresh gas and anesthetic agent to the lungs of the patient during a medical operation. This kind of device or option is an extra investment and is not necessarily available, when the situation in the operating room requires its use, and requires an extra connection to oxygen supply. Also delivering 100 % oxygen to the patient arise a series of problems, which are increased risk for fire, risk of atelectasis of the lungs and suppression of the respiration.

Another alternative used by caregivers is to use a common fresh gas outlet with the gas delivery device of the anesthesia machine to deliver an oxygen-air mixture with an oxygen concentration lower than 100%. Oxygen concentrations used are typically 25 - 50% the balance being nitrogen. Using the common fresh gas outlet as the source for supplemental oxygen has the potential hazard of delivery of a gas mixture including inhaled anesthetic agent such as halothane, enflurane, isoflurane, desflurane, sevoflurane or nitrous oxide in mistake due to a user error.

US 5049317 discloses an arrangement for generating and metering a gas mixture. The gas from gas sources can be guided through throttle elements and shutoff valves and further along a bypass line past a vaporizer and along a vaporizer line through the vaporizer to a consumer. The flow from the gas sources through the bypass line and the vaporizer line is controlled by a shutoff elements.

US 2011/0197889 discloses an apparatus and method to supply a breathing gas for a respiration. The apparatus comprises a reciprocating unit receiving inspiration gas flow and controlling respiratory movements, a gas mixer for supplying a fresh gas flow, and a breathing circuit conducting an expiration gas flow to the reciprocating unit, conducting the fresh gas flow from the gas mixer for the respiration and conducting the gas flow from the reciprocating unit for an inspiration. The apparatus also comprises a bypass passage permitting bypassing the reciprocating unit and connecting the breathing circuit to the inspiration gas flow upstream from the reciprocating unit.

US 2006/196505 discloses a portable anesthesia machine comprising a chassis with a cradle to receive a vaporizer having a vaporizer input and outlet, a control panel and a carrying support member. The control panel comprises a gas flow path with gas inputs for carrier gases, a manifold combining the gases, a carrier gas outlet and inlet being connectable to the vaporizer input and outlet, respectively, a common port for a patient cannula, and a flow control devices to control the flow rate of the carrier gases. A controller is provided to control the gas flow into and around the vaporizer to maintain constant concentration of anesthetic agent in the gas delivered to the patient.

EP 0916357 discloses a method and arrangement to supply fresh gas to a patient and to supply part of the patient's breathing gas to the patient with bellows. The fresh gas is supplied to the patient by means of a gas mixer and the fresh gas flow from the gas mixer is occasionally led to drive the bellows.

### BRIEF DESCRIPTION OF THE INVENTION

The above-mentioned shortcomings, disadvantages and problems are addressed herein which will be understood by reading and understanding the following specification.

In an embodiment, a gas delivery unit for delivering breathing gas for subject breathing includes a first regulating valve for regulating a first gas flow, and a second regulating valve for regulating a second gas flow. The gas delivery unit for delivering breathing gas also includes a first flow measurement device in flow communication with the first regulation valve for providing a signal indicative of the first gas flow and a second flow measurement device in flow communication with the second regulation valve for providing a signal indicative of the second gas flow. The gas delivery unit for delivering breathing gas also includes at least one anesthetic agent supply for supplying a mixture of an anesthetic agent and at least one of the first and second gas flows, said mixture being adapted to be delivered to a breathing circuit for subject breathing, and a valve unit being in flow communication with at least one of the first flow measurement device and the second flow measurement device for delivering gas for subject breathing. The valve unit comprises a first output for delivering the gas along a first output channel bypassing the anesthetic agent supply, which first output channel is in flow communication with an auxiliary gas outlet attachable in front of respiratory passages of a subject, and a second output for delivering the gas along a second output channel to the anesthetic agent supply for supplying said mixture of anesthetic agent and gas, said valve unit being adapted to select either said first output or said second output.

Various other features, objects, and advantages of the invention will be made apparent to those skilled in art from the accompanying drawings and detailed description thereof.

### BRIEF DESCRIPTION OF THE DRAWINGS

Figure 1 is a schematic view of an arrangement comprising a gas delivery unit in accordance with an embodiment; and
Figure 2 is a schematic view of an arrangement comprising a gas delivery unit in accordance with another embodiment.

### DETAILED DESCRIPTION OF THE INVENTION

Specific embodiments are explained in the following detailed description making a reference to accompanying drawings. These detailed embodiments can naturally be modified and should not limit the scope of the invention as set forth in the claims.

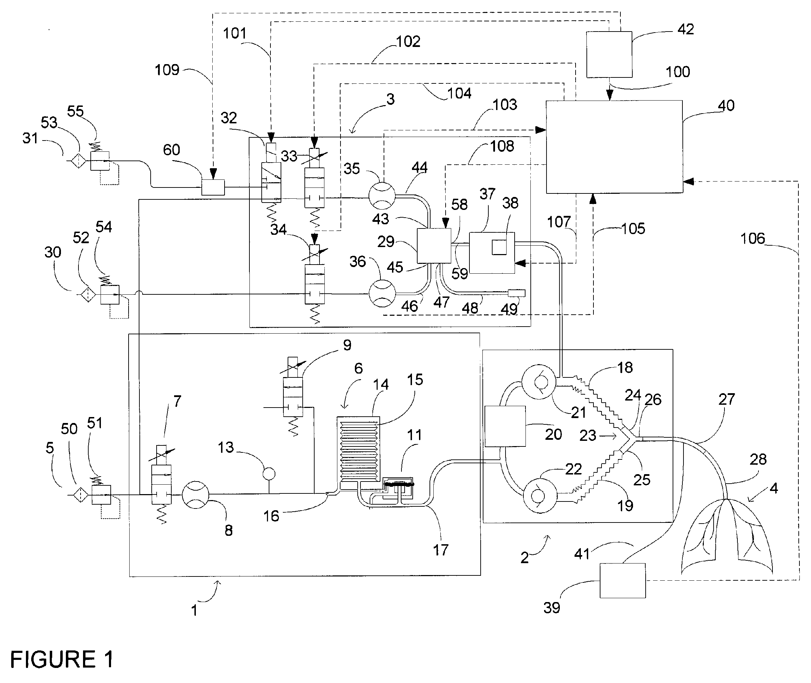

Figure 1 shows an arrangement for maintaining vital functions of a subject including delivering breathing gas which may include as an option an anesthetic agent. The arrangement may comprise a gas delivery unit 3, a ventilator 1 and a breathing circuit 2 connecting lungs of a subject 4 with the gas delivery unit 3 and ventilator 1 to exchange the gas in the lungs and a controller 40 for controlling these functions. The arrangement also may comprise a gas analyzer 39 and a user interface 42The controller may also be part of the gas delivery unit. The subject 4 is connected to the breathing circuit 2 by means of an endotracheal tube 28.

In Figure 1 the ventilator 1 is connected to a first gas supply 5, which is typically pressurized air. The gas supply 5 is operably connected to filtering 50 and ventilator pressure regulation 51. The ventilator 1 may comprise a reciprocating unit 6 for compressing gas towards lungs of the subject to facilitate the inspiration, a flow control valve 7 to control the inspired gas flow from the first gas supply 5 towards the reciprocating unit 6, an inspiration flow sensor 8 for measuring a ventilator gas flow added for the inspiration, which flow sensor typically locates between the flow control valve 7 and the reciprocating unit 6. Further the ventilator 1 may comprise an expiration valve 9 used to control the expired gas flow rate releasing the gas of the ventilator 1 when the subject 4 is expiring. The ventilator 1 can also comprise a scavenging valve 11 allowing an extra expired gas of the subject 4 to leave the breathing circuit 2. Very often the ventilator 1 also comprises a ventilator pressure sensor 13 to measure a pressure of the ventilator gas upstream the reciprocating unit 6 and the ventilator 1 may be equipped with a gas supply selection (not shown) that can be used to switch the ventilator gas flow for the inspiration either manually or automatically e.g. in case the used gas gets un-pressurized. The reciprocating unit 6 shown in Figure 1 comprises a bottle 14 and a bellows 15 within the bottle 14 for controlling respiratory movements of the subject's lungs.

When ventilating the subject, the expiration valve 9 is closed and the flow control valve 7 is opened for the inspiration flow. This flow fills the bottle 14 making the bellows 15 to push down pushing the gas within the bellows further towards the subject 4. During the expiration the flow control valve 7 is closed and the expiration valve 9 is opened to control the expiration flow and pressure. The gas pressurized in the bottle 14 is released allowing the gas from lungs to fill the bellows 15 up. When the bellows 15 is filled, it hits the top of the bottle 14, and the further gas flow into the system increases the pressure within the bellows 15. When this pressure exceeds the bottle pressure, the scavenging valve 11 will open allowing the further gas flow to scavenging valve 11.

The ventilator 1 is operably connected to the breathing circuit 2 such as a re-breathing circuit by means of a ventilator tube 17 for both inspired and expired gas flows. The breathing circuit 2 comprises an inspiration tube 18 for inspired gas, an expiration tube 19 for expired gas, a CO2 remover 20 such as CO2 absorber to remove or absorb carbon dioxide from the exhaled gas coming from the subject 4, a first one way valve 21 to allow an inspiration through the inspiration tube 18, a second one way valve 22 to allow an expiration through the expiration tube 19, a branching unit 23 such as a Y-piece having at least three limbs, one of them being an inhalation limb 24 for inspired gas, a second one being an expiration limb 25 for expired gas, a third one being a combined inspiration and expiration limb 26 for both inspired and expired gases. The inhalation limb 24 is connectable to the inspiration tube 18 and the expiration limb 25 is connectable to the expiration tube 19. The combined inspiration and expiration limb 26 of the branching unit 23 may be connectable by means of a patient tube 27 to the endotracheal tube 28 allowing the gas exchange with airways of the subject 4.

The inspiration gas flows from the reciprocating unit 6 through the ventilator tube 17, the CO2 remover 20 and the inspiration tube 18 of the breathing circuit 2 to the branching unit 23 and further through the patient tube 27 and the endotracheal tube 28 to the lungs of the subject 4. The second one-way valve 22 on the expiratory tube 19 guides the gas flow direction to the inspiration tube 18 by closing the flow from the ventilator tube 17 through the expiration tube 19. Increasing the gas volume within the lungs increases the lung pressure due to the lung compliance. Once the inspiration stops and the expiration begins the expiration valve 9 opens relieving the bottle 14 pressure, the lung compliance pushes the alveolar gas through the endotracheal tube 28 and the patient tube 27 to the branching unit 23 and further through the expiration tube 19 and the ventilator tube 17 to fill the bellows 15.

The gas delivery unit 3 for delivering a fresh gas is operably connected to the breathing circuit 2. The gas delivery unit 3 is used to form the subject breathing gas. One or more gas supplies 5, 30, 31 may be connected to the gas delivery unit 3. A first gas flow is supplied from the first gas supply 5 and the second gas flow is supplied from at least one of a second gas supply 30 and a third gas supply 31. The second gas supply 30 and the third gas supply 31 are just as the first gas supply 5 operably connected to respective filterings 52, 53 and pressure regulations 54, 55 for supplying the gas. The first gas supply 5 is for the air as described above including a balance gas such as nitrogen, the second gas supply 30 is for oxygen and the third gas supply 31 is for an alternate balance gas, which is typically nitrous oxide. Nitrous oxide is a narcotic agent. The gas delivery unit comprises a selector valve 32 to select either the third gas supply 31 for nitrous oxide or the first gas supply 5 for air, a first flow regulating valve 33 for adjusting the first gas flow, a second flow regulating valve 34 for adjusting second gas flow which is in this case oxygen flow and an at least one anesthetic agent supply 37 such as a vaporizer for supplying in case of need an anesthetic agent to anesthetize the subject 4. The gas delivery unit 3 also comprises a first flow measurement device 35, such as a flow sensor or rotameter, in flow communication with the first regulating valve 33 for providing a signal indicative of the first gas flow comprising at least one of air delivered from the gas supply 5 and nitrous oxide delivered from the third gas supply 31 or comprising their mixture and which second gas is delivered through the first regulating valve 33 to the first flow measurement device 35 based on which flow signal the individual first gas flow can be determined. The signal indicative of the first gas flow can be for example electrical or which can be read from the scale of the measurement device. The gas delivery unit 3 further comprises a second flow measurement device 36, such as a flow sensor or rotameter, in flow communication with the second regulating valve 34 for providing a signal indicative of the second gas flow comprising at least oxygen or advantageously substantially oxygen, typically 90-100% oxygen delivered from the second gas supply 30 and which second gas is delivered through the second regulating valve 34 to the second flow measurement device 36 based on which flow signal the individual second gas flow can be determined. The signal indicative of the second gas flow can be for example electrical or which can be read from the scale of the measurement device. Both the first and second gas flow may be added into the breathing circuit 2 for respiration. Typically the first and second flow measurement devices 35, 36 are downstream the corresponding first and second flow regulating valves 33, 34, but their order may be different, too.

The flow determination of the first and second gas may be made based on flow signals received by the controller 40, when the first flow measurement device 35 provides the signal indicative of the first gas flow to the controller 40 and the second flow measurement device 36 provides the signal indicative of the second gas flow to the controller 40. The controller is comparing the first and second fresh flow with a required gas flow information after which the controller 40 may regulate the first regulating valve 33 and the second regulating valve 34 or only one of them to deliver the required gas flows when required, which is typically when the determined flow results deviate substantially from the required gas flows. After the first and second flow measurement devices the individual gas flows are usually merged to a gas mixture upstream the anesthetic agent supply 37. The mixture may then be further guided to join with the anesthetic agent vaporized in the anesthetic agent supply 37 for adding the anesthetic inhalation agent into the mixture as shown in Figure 1. There may be one or more anesthetic agent supplies, but according to the existing device requirements only one anesthetic agent supply is allowed to be active for the vaporization at a time. To adjust the anesthetic agent vaporized by means of the anesthetic agent supply 37 an actuator 38 is needed. Especially in the closed loop anesthetic agent control the actuator 38 can be used for automatic control of the anesthetic agent delivery by means of the controller 40. The anesthetic agent can also be injected directly into the breathing circuit 2 in a liquid or vaporized form by means of the anesthetic agent supply 37 when it will be vaporized to the gas in the circuit or as a vapor.

In Figure 1 there is further a valve unit 29, such as a switch valve, which is part of the gas delivery unit 3 in this embodiment, and which is needed for supplying supplemental oxygen bypassing the anesthetic agent supply 37 for the subject breathing for example during local and regional anesthesia. The valve unit is upstream the anesthetic agent supply 37, which valve unit is between the anesthetic agent supply 37 and the gas supplies 5, 30, 31, more specifically between the anesthetic agent supply 37 and the regulation valves 33, 34, or even more specifically between the anesthetic agent supply 37 and the flow measurement devices 35, 36. The valve unit 29 comprises a first input 43 for receiving the first gas flow along a first input channel 44 from at least the first gas supply 5 for supplying air and, if desired, from the third gas supply 31 for supplying nitrous oxide, but typically nitrous oxide is not delivered when bypassing the anesthetic agent supply 37 and supplemental oxygen is required for the subject breathing. The valve unit 29 also comprises a second input 45 for receiving the second gas flow along a second input channel 46 from the second gas supply 30 for supplying oxygen.

The valve unit 29 comprises further a first output 47 for delivering the gas, which may be a mixture of the first and second gas flow, along a first output channel 48 bypassing the anesthetic agent supply 37 and typically bypassing the breathing circuit 2, too. Through the first output channel 48 the gas including oxygen, which may be one of substantially pure oxygen and a mixture of oxygen and air, is delivered to the subject breathing bypassing the anesthetic agent supply 37 to avoid the supply of anesthetic agent and to make sure that the subject is receiving only anesthetic agent free gas and which mixture is not advantageously so flammable as only pure oxygen. So the first output channel 48 is in flow communication with an auxiliary gas outlet 49 attachable in front of respiratory passages of the subject. The auxiliary gas outlet is connectable to a breathing mask, such as face mask, or nasal cannula.

As shown in Figure 1 the valve unit 29 may also comprise a second output 58 for delivering the gas along a second output channel 59 to the anesthetic agent supply 37 for supplying anesthetic agent in case of need, which is further connected to the breathing circuit 2 for supplying a mixture of anesthetic agent and the fresh gas to the lungs of the subj ect.

Thus the valve unit 29 enables to select between two different gas delivery paths in case of need. One of those two paths is for mixing the anesthetic agent supplied by the anesthetic agent supply 37 with gas delivered through at least one of the first flow measurement device 35, the first regulation valve 33, the second flow measurement device 36 and the second regulation valve 34. This mixture is typically delivered when anesthetizing the subject or when maintaining the desired level of anesthesia. Another of those two paths is for the gas delivered through at least one of the first flow measurement device, the first regulation valve, the second flow measurement device and the second regulation valve bypassing the anesthetic agent supply. Typically the anesthetic agent supply is bypassed when the subject needs supplemental oxygen.

The valve unit 29 is controlled by the controller 40 for example based on an instruction set by a user on the user interface 42 and received by the controller from the user interface 42. The controller 40 is able to select one of the first output and the second output for gas delivery. The first output 47 is opened by the controller 40 for delivering the fresh gas including oxygen to the subject breathing and the first output 47 is closed for discontinuing or interrupting the delivery of the fresh gas. The opening and closing operation can also be done manually, if desired. In case the valve unit 29 comprises the second output 58, it can be also controlled by the controller 40 or it can be done manually. The second output 58 can be closed if only the gas through the first output 47 should be delivered to the subject or the second output 58 can be opened if the gas delivery through the anesthetic agent supply 37 is selected in which case typically the gas delivery through the first output is discontinued.

The controller 40 can use the signal indicative of the first gas flow obtained from the first flow measurement device 35 to regulate the first regulating valve 33 and the signal indicative of the second gas flow obtained from the second flow measurement device 36 to regulate the second regulating valve 34 to deliver the required gas flow through the first output 47 of the switch valve 29 and along the first output channel 48 bypassing the anesthetic agent supply 37. Also the controller 40 may discontinue nitrous oxide delivery from the gas supply 31 by using a selector valve 32 to select air from the gas supply 5. There can advantageously be a shut-off valve 60 to discontinue the nitrous oxide flow to the first output channel 48 and which valve can be either manually or electrically controlled by the controller 40. The user may set the desired gas component and concentration through the user interface 42 to the controller 40 or the controller 40 may do this selection based on the information set in the factory.

The gas analyzer 39 is enabled to measure at least one compound of the breathing gas including inhalation and/or exhalation gas concentrations such as the end-tidal concentrations of one or more anesthetic agent and nitrous oxide and possibly oxygen. The gas analyzer 39 may also identify e.g. anesthetic agents and nitrous oxide in case they are not known. The gas analyzer 39 of Figure 1 is a side-stream analyzer equipped with a mechanism that takes a sample of the breathing gas through a sampling line 41 for analysis within the gas analyzer. This sample gas flow is 50-250 mL/min depending on the analyzer. The gas analyzer 39 may as an option (not shown in Figure) also measure at least one of at least one gas components and gas component concentration of the gas including oxygen flowing along the first output channel 48 towards the subject. Alternatively the gas analyzer 39 could be a mainstream type connected directly to the breathing circuit 2, the patient tube 27 or the first output channel 48 without any sampling line 41. In either case gas analyzers are nowadays typically based on infrared absorption technique.

The controller 40 includes a processing unit (not shown in the Figure) to receive the information indicative of the gas flow, which may be a mixture of the first and second gas flows, along the first output channel 48 and/or the measured breathing gas concentration of the anesthetic agent from the anesthetic agent supply 37 and/or nitrous oxide from the third gas supply 31 delivered through the breathing circuit 2 to the subject. Through the user interface 42 along a signal line 100 the user may set to the controller 40 one or more desired target values indicative of one or more gas component concentrations.

To take into consideration all anesthetic agents and nitrous oxide of the breathing gas the controller 40 is able to collect from the gas analyzer 39 the information indicative of measured breathing gas concentration of each compound and compare the desired target value received from the user interface 42 and determine whether or not to change the gas flow, nitrous oxide and anesthetic agent supply. In case the desired target value deviates from measurement results of the compounds the actuator 38 is under the control of the controller 40 adjusting the anesthetic agent flow from the narcotic agent supply 37 in order to reduce a deviation. The user interface 42 can be used to inform the controller 40 about various facts useful while administering various gases for the subject breathing.

The controller 40 is connected through a signal line 101 to the selector valve 32 of the gas delivery unit 3, which signal line 101 is adapted to carry a signal from the controller 40 to the selector valve 32. Also the controller 40 is connected through a signal line 102 to the flow regulating valve 33 of the fresh gas delivery unit, which signal line 102 is adapted to carry a signal from the controller 40 to the flow regulating valve 33. Further the controller 40 is connected through a signal line 103 to the first flow measurement device 35 of the fresh gas delivery unit 3, which signal line 103 is adapted to carry a signal from this first flow measurement device 35 to the controller 40. Also the controller 40 is connected through a signal line 104 to the second regulating valve 34 of the gas delivery unit 3, which signal line 104 is adapted to carry a signal from the controller 40 to the flow regulating valve 34. This controller 40 is also connected through a signal line 105 to the second flow measurement device 36, which signal line 105 is adapted to carry a signal from the second flow measurement device 36 to the controller 40. The controller 40 is also connected through a signal line 106 to the gas analyzer 39, which signal line is adapted to carry a signal indicative of a measured alveolar concentration of each narcotic agent and/or a signal indicative of a measured fresh gas including oxygen concentration and gas components of the fresh gas from the gas analyzer 39 to the controller 40. The signal line 107 is needed in inhalation anesthesia to carry a signal between the actuator 38 for at least one narcotic agent supply 37 and the controller 40 to adjust the narcotic agent. A signal line 108 is used to carry a signal from the controller 40 to the valve unit 29. A signal line 109 is used to carry signal from the controller 40 to the shut-off valve 60.

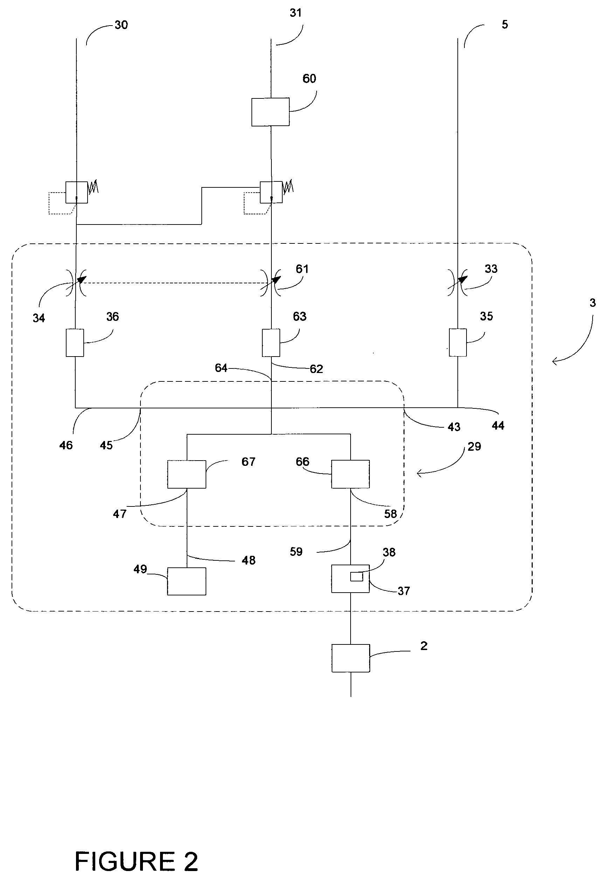

Figure 2 shows another embodiment of the gas delivery unit 3 having a separate flow path for a third gas flow, which in this embodiment is nitrous oxide supplied by the third gas supply 31, and which separate flow path extends to the valve unit 29. This flow path comprises a shut-off valve 60 as shown in the previous embodiment, too, but without the selector valve 32, because air from the first gas supply 5 has its own flow path with the first flow measurement device 35 and the first regulating valve 33. The flow path from the gas supply 31 for nitrous oxide is provided with a third regulating valve 61 for regulating the third gas flow and a third flow measurement device 63, such as a flow sensor or rotameter, for acquiring a signal indicative of the third gas flow.

Also the gas delivery unit 3 shown in Figure 2 comprises the valve unit 29 having a third input 64 receiving the third gas flow along a third input channel 62. The first, second and third gas flows are mixed inside the valve unit 29, but as well the first input channel 44, the second input channel 46 and the third input channel 62 could join outside the valve unit 29 mixing the gases, in which case the mixed gas may be conveyed through a single channel to the valve unit through only one physical input, which may be one of the first input 45, second input 45 and third input 64, but it should be understood that for functional reasons this single input can also be seen to comprise at least the first input and the second input.

The gas flow path inside the valve unit 29 is divided into two different flow paths with separate shut-off valves 66 and 67. The shut-off valve 66 is used to control the delivery of the mixed gas flow through the second output 58 to the anesthetic agent supply 37 and further to the breathing circuit 2 before reaching lungs of the subject 4. Another shut-off valve 67 is used to control the delivery of mixed gas flow through the first output 47 to the first output channel 48 and further to the auxiliary gas outlet 49. The shut-off valves 66, 67 typically are able either to discontinue the gas delivery or to allow the gas delivery. Various components of the gas delivery unit 3 can be controlled by the controller 40 similarly as explained with the embodiment in Figure 1. Also it should be noted that various components can be controlled manually as well.

These embodiments provide ease of use when changing from anesthetic agent delivery to the gas delivery including either substantially pure oxygen or advantageously oxygen-air mixture without anesthetic agent compound and in most cases without nitrous-oxide. The same gas delivery unit as known before provided with the valve unit 29 is an inexpensive solution enabling to change from anesthetic agent delivery to the supplemental oxygen delivery through the auxiliary gas outlet 49 with a variable oxygen concentration. The embodiment also provides a patient safety in preventing accidental anesthetic agent delivery when changing to the supplemental oxygen delivery and reduction of risk for fire when using a gas mixture with less than 100% oxygen concentration.

The written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A gas delivery unit for delivering breathing gas for subject breathing comprising:
a first regulating valve (33) for regulating a first gas flow;
a second regulating valve (34) for regulating a second gas flow;
a first flow measurement device (35) in flow communication with said first regulation valve for providing a signal indicative of the first gas flow;
a second flow measurement device (36) in flow communication with said second regulation valve for providing a signal indicative of the second gas flow; and
at least one anesthetic agent supply (37) for supplying a mixture of an anesthetic agent and at least one of the first and second gas flows, said mixture being adapted to be delivered to a breathing circuit (2) for subject breathing;
**characterized in that** the gas delivery unit also comprises a valve unit (29) in flow communication with at least one of said first flow measurement device and said second flow measurement device for delivering gas for subject breathing, said valve unit comprising a first output (47) for delivering the gas along a first output channel (48) bypassing said anesthetic agent supply, which first output channel is in flow communication with an auxiliary gas outlet (49) attachable in front of respiratory passages of a subject, and a second output (58) for delivering the gas along a second output channel (59) to the anesthetic agent supply (37) for supplying said mixture of anesthetic agent and gas, said valve unit (29) being adapted to select either said first output (47) or said second output (58).

2. The gas delivery unit according to claim 1, **characterized in that** said valve unit comprises at least one of a first input (43) in flow communication with said first flow measurement device and said first regulating valve delivering the first gas flow and a second input (45) in flow communication with said second flow measurement device and said second regulating valve delivering the second gas flow, and said first output (47) being adapted for delivering at least one of the first gas received from said first input and the second gas received from said second input for subject breathing bypassing said anesthetic agent supply.

3. The gas delivery unit according to claim 2, **characterized in that** said first input of said valve unit is adapted to be in flow communication with said first flow measurement device along said first input channel (44), said second input of said valve unit is adapted to be in flow communication with said second flow measurement device along a second input channel (46).

4. The gas delivery unit according to claim 3, **characterized in that** said an auxiliary gas outlet (49) is connectable to a breathing mask or nasal cannula.

5. The gas delivery unit according to claim 1, further comprising a selector valve (32) adapted to select between at least two gas supplies, which are a third gas supply (31) and a first gas supply (5), and which selector valve is adapted to be in flow communication with said first regulation valve and said first flow measurement device, and also comprising as an option a shut-off valve (60) to discontinue the gas delivery from said third gas supply.

6. The gas delivery unit according to claim 5, **characterized in that** said first gas supply is for air and said third gas supply is for nitrous oxide.

## Patentansprüche

1. Gaszufuhreinheit zum Zuführen von Atemgas für das Atmen eines Subjekts, Folgendes umfassend:
ein erstes Regulierventil (33) zum Regulieren eines ersten Gasstroms;
ein zweites Regulierventil (34) zum Regulieren eines zweiten Gasstroms;
ein erstes Strommessgerät (35) in Strömungsverbindung mit dem ersten Regulierventil zum Bereitstellen eines Signals, das den ersten Gasstrom anzeigt;
ein zweites Strommessgerät (36) in Strömungsverbindung mit dem zweiten Regulierventil zum Bereitstellen eines Signals, das den zweiten Gasstrom anzeigt; und
wenigstens eine Anästhesiemittelzuleitung (37) zum Zuleiten einer Mischung eines Anästhesiemittels und des ersten und/oder des zweiten Gasstroms, wobei die Mischung angepasst ist, um einem Atemkreislauf (2) zum Atmen eines Subjekts zugeleitet zu werden.
**dadurch gekennzeichnet, dass** die Gaszufuhreinheit auch eine Ventileinheit (29) umfasst, in Strömungsverbindung mit dem ersten Strommessgerät und/oder dem zweiten Strommessgerät zum Zuführen von Gas zum Atmen eines Subjekts, wobei die Ventileinheit einen ersten Ausgang (47) zum Zuführen des Gases entlang eines ersten Ausgangskanals (48) umfasst, der die Anästhesiemittelzuleitung umgeht, wobei der erste Ausgangskanal in Strömungsverbindung mit einem zusätzlichen Gasauslass (49) steht, der vor den Atemwegen eines Subjekts befestigt werden kann, und einen zweiten Ausgang (58) zum Zuführen des Gases entlang eines zweiten Ausgangskanals (59) zur Anästhesiemittelzuleitung (37) zum Zuleiten der Mischung des Anästhesiemittels und des Gases, wobei die Ventileinheit (29) angepasst ist, um entweder den ersten Ausgang (47) oder den zweiten Ausgang (58) auszuwählen.

2. Gaszufuhreinheit nach Anspruch 1, **dadurch gekennzeichnet, dass** die Ventileinheit Folgendes umfasst: einen ersten Eingang (43), der mit dem ersten Strommessgerät und dem ersten Regulierventil, das den ersten Gasstrom zuführt, in Strömungsverbindung steht, und/oder einen zweiten Eingang (45), der mit dem zweiten Strommessgerät und dem zweiten Regulierventil, das den zweiten Gasstrom zuführt, in Strömungsverbindung steht, und wobei der erste Ausgang (47) angepasst ist für das Zuführen des ersten Gases, das vom ersten Eingang empfangen wurde, und/oder des zweiten Gases, das vom zweiten Eingang empfangen wurde, zum Atmen eines Subjekts, wobei die Anästhesiemittelzuleitung umgangen wird.

3. Gaszufuhreinheit nach Anspruch 2, **dadurch gekennzeichnet, dass** der erste Eingang der Ventileinheit angepasst ist, um mit dem ersten Strommessgerät entlang des ersten Eingangskanals (44) in Strömungsverbindung zu stehen, wobei der zweite Eingang der Ventileinheit angepasst ist, um mit dem zweiten Strommessgerät entlang eines zweiten Eingangskanals (46) in Strömungsverbindung zu stehen.

4. Gaszufuhreinheit nach Anspruch 3, **dadurch gekennzeichnet, dass** der zusätzliche Gasauslass (49) mit einer Atemmaske oder einer Nasenkanüle verbunden werden kann.

5. Gaszufuhreinheit nach Anspruch 1, ferner umfassend ein Wahlventil (32), das angepasst ist, um zwischen wenigsten zwei Gaszuleitungen auszuwählen, die eine dritte Gaszuleitung (31) und eine erste Gaszuleitung (5) sind, und wobei das Wahlventil angepasst ist, mit dem ersten Regulierventil und dem ersten Strommessgerät in Strömungsverbindung zu stehen, und außerdem umfassend als eine Option ein Absperrventil (60), um die Gaszufuhr von der dritten Gaszuleitung abzubrechen.

6. Gaszufuhreinheit nach Anspruch 5, **dadurch gekennzeichnet, dass** die erste Gaszuleitung für Luft ist und die dritte Gaszuleitung für Distickstoffmonoxid ist.

## Revendications

1. Unité de fourniture de gaz pour fournir un gaz respiratoire pour la respiration d'un sujet, comprenant :
une première vanne de régulation (33) pour réguler un premier écoulement de gaz ;
une seconde vanne de régulation (34) pour réguler un second écoulement de gaz ;
une premier dispositif de mesure de débit (35) en communication fluidique avec ladite première vanne de régulation pour fournir un signal indicatif du premier écoulement de gaz ;
un second dispositif de mesure de débit (36) en communication fluidique avec ladite seconde vanne de régulation pour fournir un signal indicatif du second écoulement de gaz ; et
au moins une source d'agent anesthésiant (37) pour fournir un mélange d'agent anesthétique et d'au moins l'un du premier et du second écoulement de gaz, ledit mélange étant à même d'être fourni à un circuit de respiration (2) pour la respiration du sujet ;
**caractérisée en ce que** l'unité de fourniture de gaz comprend une unité de vanne (29) en communication fluidique avec au moins l'un dudit premier dispositif d'écoulement et dudit second dispositif d'écoulement pour fournir du gaz pour la respiration du sujet, ladite unité de vanne comprenant une première sortie (47) pour fournir le gaz le long d'un premier canal de sortie (48) contournant ladite source d'agent anesthétique, lequel premier canal de sortie est en communication fluidique avec une sortie de gaz auxiliaire (49) fixable devant des passages respiratoires d'un sujet, et une seconde sortie (58) pour fournir le gaz le long d'un second canal de sortie (59) à la source d'agent anesthétique (37) afin de fournir ledit mélange d'agent anesthétique et de gaz, ladite unité de vanne (29) étant à même de choisir ladite première sortie (47) ou ladite seconde sortie (58).

2. Unité de fourniture de gaz selon la revendication 1, **caractérisée en ce que** ladite unité de vanne comprend au moins l'une d'une première entrée (43) en communication fluidique avec ledit premier dispositif de mesure de débit et ladite première vanne de régulation fournissant le premier écoulement de gaz et d'une seconde entrée (45) en communication fluidique avec ledit second dispositif de mesure d'écoulement et ladite seconde vanne de régulation fournissant le second écoulement de gaz, ladite première sortie (47) étant à même de fournir au moins l'un du premier gaz reçu de ladite première entrée et du second gaz reçu de ladite seconde entrée pour la respiration du sujet contournant ladite source d'agent anesthétique.

3. Unité de fourniture de gaz selon la revendication 2, **caractérisée en ce que** ladite première entrée de ladite unité de vanne est à même d'être en communication fluidique avec ledit premier dispositif de mesure de débit le long dudit premier canal d'entrée (44), tandis que la seconde entrée de ladite unité de vanne est à même d'être en communication fluidique avec ledit second dispositif de mesure de débit le long d'un second canal d'entrée (46).

4. Unité de fourniture de gaz selon la revendication 3, **caractérisée en ce que** ladite une sortie de gaz auxiliaire (49) peut être raccordée à un masque respiratoire ou à une canule nasale.

5. Unité de fourniture de gaz selon la revendication 1, comprenant en outre une vanne sélectrice (32) qui est à même de choisir entre au moins deux sources de gaz, qui sont une troisième source de gaz (31) et une première source de gaz (5), laquelle vanne sélectrice est à même d'être en communication fluidique avec ladite première vanne de régulation et ledit premier dispositif de mesure de débit, et comprenant également en option une vanne d'arrêt (60) pour interrompre la fourniture de gaz en provenance de ladite troisième source de gaz.

6. Unité de fourniture de gaz selon la revendication 5, **caractérisée en ce que** ladite première source de gaz est pour de l'air et ladite troisième source de gaz est pour de l'oxyde nitreux.
